# EUROPEAN PATENT APPLICATION

(11) **EP 1 975 147 A1**
(43) Date of publication of application: **01.10.2008**
(21) Application number: 06843118.8
(22) Date of filing: 19.12.2006
(51) Int. Cl.: C07C 43/174, C07C 17/093, C07C 17/16, C07C 25/13, C07C 29/10, C07C 33/46, C07C 41/18, C07C 43/225, C07B 61/00

(54) **TETRAFLUOROTOLUENE COMPOUND, METHOD FOR PRODUCING SAME AND USE THEREOF**

(30) Priority: 22.12.2005 JP 2005369277
(71) Applicant: Sumitomo Chemical Company, Limited, Tokyo 104-8260 (JP)
(72) Inventor: HAGIYA, Koji, Ibaraki-shi, Osaka 567-0833 (JP)
(74) Representative: Duckworth, Timothy John
(86) International application number: PCT/JP2006/325688
(87) International publication number: WO 2007/072966

(57) **Abstract**

A tetrafluorotoluene compound represented by the formula (1): 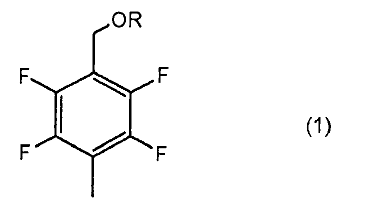 wherein R represents a C1-C6 alkyl group, and a method for producing a tetrafluorotoluene compound represented by the formula (1) which comprises hydrogenating a tetrafluorobenzyl alcohol compound represented by the formula (2): 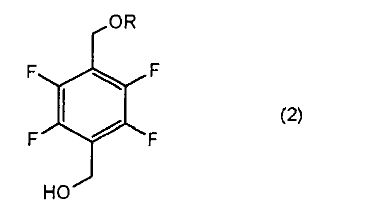 Wherein R represents a C1-C6 alkyl group.

## Description

### Technical Field

The present invention relates to a tetrafluorotoluene compound, a method for producing the same and a use thereof.

### Background Art

4-Methyl-2,3,5,6-tetrafluorobenzyl alcohol, 4-methyl-2,3,5,6-tetrafluorobenzyl bromide and 4-methyl-2,3,5,6-tetrafluorobenzyl acetate are useful as synthetic intermediates of pharmaceuticals and agrichemicals (e.g. US Patent No. 6225495 and GB Patent No. 2123824).

As a method for producing 4-methyl-2,3,5,6-tetrafluorobenzyl alcohol, JP 2002-173455, and A discloses a method comprising hydrogenating 2,3,5,6-tetrafluorobenzene-1,4-dimethanol, and JP 2004-512319 A and CN 1458137 A disclose a method comprising brominating 2,3,5,6-tetrafluorobenzene-1,4-dimethanol followed by reduction, respectively.

As a method for producing 4-methyl-2,3,5,6-tetrafluorobenzyl bromide, JP 59-130822 A discloses a method comprising brominating 2,3,5,6-tetrafluoro-p-xylene, and JP 2003-238458 A discloses a method comprising brominating 4-methyl-2,3,5,6-tetrafluorobenzyl alcohol.

As a method for producing 4-methyl-2,3,5,6-tetrafluorobenzyl acetate, GB 2123824 B discloses a method comprising contacting 2,3,5,6-tetrafluoro-p-xylene with oxygen in the presence of cobalt acetate, sodium acetate, sodium bromide, acetic acid and acetic anhydride.

### Disclosure of the Invention

The present invention provides a tetrafluorotoluene compound represented by the formula (1): 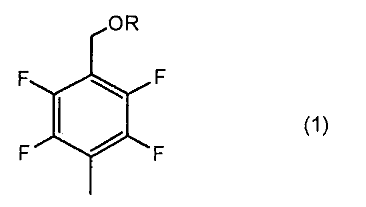 wherein R represents a C1-C6 alkyl group,
a method for producing the tetrafluorotoluene compound represented by the above-mentioned formula (1) comprising hydrogenating a tetrafluorobenzyl alcohol compound represented by the formula (2): 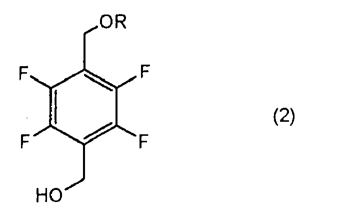 wherein R represents a C1-C6 alkyl group,
a method for producing 4-methyl-2,3,5,6-tetrafluorobenzyl acetate comprising reacting a tetrafluorotoluene compound represented by the represented by the above-mentioned formula (1), and a method for producing 4-methyl-2,3,5,6-tetrafluorobenzyl bromide comprising reacting a tetrafluorotoluene compound represented by the represented by the above-mentioned formula (1).

### Best Mode for Carrying Out the Present Invention

First, a novel compound, a tetrafluorotoluene compound represented by the formula (1): 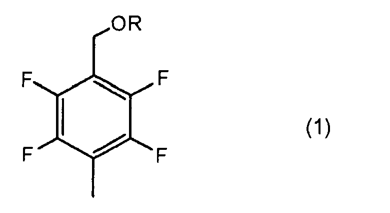 wherein R represents a C1-C6 alkyl group (hereinafter, simply referred to as the tetrafluorotoluene compound (1)), will be illustrated.

Examples of the C1-C6 alkyl group include a linear, branched chain or cyclic alkyl group such as a methyl group, an ethyl group, an n-propyl group, an isopropyl group, an n-butyl group, an isobutyl group, a sec-butyl group, a tert-butyl group, an n-pentyl group, a cyclopentyl group and a cyclohexyl group.

Examples of the tetrafluorotoluene compound (1) include 4-methoxymethyl-2,3,5,6-tetrafluorotoluene, 4-ethoxymethyl-2,3,5,6-tetrafluorotoluene, 4-n-propoxymethyl-2,3,5,6-tetrafluorotoluene, 4-isopropoxymethyl-2,3,5,6-tetrafluorotoluene, 4-n-butoxymethyl-2,3,5,6-tetrafluorotoluene, 4-isobutoxymethyl-2,3,5,6-tetrafluorotoluene and 4-n-hexyloxymethyl-2,3,5,6-tetrafluorotoluene.

The tetrafluorotoluene compound (1) can be produced by hydrogenating a tetrafluorobenzyl alcohol compound represented by the formula (2): 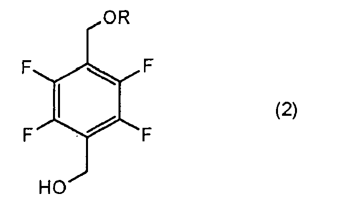 wherein R represents the same meaning as defined above (hereinafter, simply referred to as the benzyl alcohol compound (2)).

Examples of the benzyl alcohol compound (2) include 4-methoxymethyl-2,3,5,6-tetrafluoro benzyl alcohol, 4-ethoxymethyl-2,3,5,6-tetrafluoro benzyl alcohol, 4-n-propoxymethyl-2,3,5,6-tetrafluoro benzyl alcohol, 4-isopropoxymethyl-2,3,5,6-tetrafluoro benzyl alcohol, 4-n-butoxymethyl-2,3,5,6-tetrafluoro benzyl alcohol, 4-isobutoxymethyl-2,3,5,6-tetrafluoro benzyl alcohol and 4-n-hexyloxymethyl-2,3,5,6-tetrafluoro benzyl alcohol.

A commercially available benzyl alcohol compound (2) may be used and one produced by a known method described in JP 2001-220360 A etc. may be used.

The hydrogenating reaction of the benzyl alcohol compound (2) is usually conducted by mixing the benzyl alcohol compound (2) with a hydrogenating agent as necessary in the presence of a catalyst.

As the hydrogenating agent, hydrogen is usually used. Hydrogen may be used alone and may be mixed with an inert gas such as nitrogen to use.

When hydrogen is used, while the hydrogen pressure is not particularly limited, it is usually a normal pressure to 0.8 MPa, and preferably a normal pressure to 0.5 MPa.

When hydrogen is used as the hydrogenating agent, a metal catalyst is usually used. Examples of the metal catalyst include a catalyst containing at least one metal atom selected from a group consisting of cobalt, iron, nickel, platinum, palladium and rhenium. Specifically, these metals and alloy of these metals are exemplified. As these metals and alloy of these metals, one supported on carrier may be used.

Alternatively, a sponge metal can be also used as the metal catalyst. Herein, "sponge metal" means a porous metal catalyst obtained by eluting a metal which is soluble in alkali or acid from alloy of a metal which is insoluble in alkali or acid such as nickel and cobalt and a metal which is soluble in alkali or acid such aluminum, silicon, zinc and magnesium with alkali or acid. Examples thereof include sponge cobalt and sponge nickel.

Among these metal catalysts, sponge metal is preferable and sponge nickel or sponge cobalt is more preferable.

When a metal or alloy is used as it is as the metal catalyst, a metal or alloy having a small particle size is preferably used. When a metal or alloy supported on carrier is used, examples of the carrier include active carbon, alumina, silica and zeolite, and in the point of availability, active carbon is preferable and in the point of reaction activity, a carrier having a small particle size is preferable.

A metal catalyst containing water may be used.

The used amount of the metal catalyst differs depending on the form thereof, and it is usually 0.1 to 150% by weight per 1 part of the benzyl alcohol compound (2).

The hydrogenating reaction of the benzyl alcohol compound (2) is usually conducted in the presence of a solvent. The solvent is not particularly limited in so far as it is an inert solvent on the hydrogenating reaction, and examples thereof include aromatic hydrocarbon solvents such as toluene, xylene and chlorobenzene; aliphatic hydrocarbon solvents such as pentane, hexane and heptane; ether solvents such as diethyl ether and methyl tert-butyl ether; ester solvents such as ethyl acetate; alcohol solvents such as methanol, ethanol, isopropanol and tert-butanol; nitrile solvents such as acetonitrile; the ionic liquid described below; water; and a mixed solvent of them.

While the used amount of the solvent is not particularly, practical amount is 0.5 to 20 parts by weight per 1 part of the benzyl alcohol compound (2) in consideration of volume efficiency or the like.

When hydrogen gas is used as the hydrogenating agent, the ionic liquid is preferably used as the solvent in the point that the reaction can be conducted under relatively low hydrogen pressure and the desired tetrafluorotoluene compound (1) is easily isolated.

In the present invention, "ionic liquid" means a salt comprising an organic cationic species and an anionic species, which has a melting point of 100 °C or less and is stable to hold liquid state until high temperature of about 300 °C.

Examples of the ionic liquid include an alkyl-substituted imidazolium salt, an alkyl-substituted pyridinium salt, a quaternary ammonium salt, a quaternary phosphonium salt and tertiary sulfonium salt. These may be used alone and may be mixed to use. At least one selected from a group consisting of the alkyl-substituted imidazolium salt, the alkyl-substituted pyridinium salt and the quaternary ammonium salt is preferable, and among them, the alkyl-substituted imidazolium salt or the quaternary ammonium salt is more preferable.

Examples of the alkyl-substituted imidazolium salt include salts which are composed of an imidazolium cation, of which at least one nitrogen atom on the imidazoline ring is bonded to an optionally substituted alkyl group, and an anionic species such as a tetrafluoroborate anion, a chloride anion, a bromide anion, an iodide anion, a hexafluorophosphate anion, a bis(perfluoroalkanesulfonyl)amide anion, an alkylcarboxylate anion and an alkanesulfonate anion. Herein, examples of the alkyl group include a C1-C8 alkyl group such as a methyl group, an ethyl group, an n-propyl group, an isopropyl group, an n-butyl group, an isobutyl group and an n-pentyl group. Examples of the substituent wherein the alkyl group may have include a C1-C8 alkoxy group such as a methoxy group and an ethoxy group; and a halogen atom such as a fluorine atom, a chlorine atom and a bromine atom. Specific examples of the alkyl group substituted with these substituents include a chloromethyl group, a fluoromethyl group, a trifluoromethyl group, a methoxymethyl group, an ethoxymethyl group, a methoxyethyl group and a methoxycarbonylmethyl group. Thus optionally subustituted alkyl group may be also bonded to a carbon atom on the imidazoline ring.

Examples of the alkyl-substituted imidazolium salt include an alkyl-substituted imidazolium tetrafluoroborate such as 1-methyl-3-methylimidazolium tetrafluoroborate, 1-methyl -3-ethylimidazolium tetrafluoroborate, 1-methyl-3-butylimidazolium tetrafluoroborate, 1-methyl-3-isobutylimidazolium tetrafluoroborate, 1-methyl-3-methoxyethylimidazolium tetrafluoroborate, 1-ethyl-3-ethylimidazolium tetrafluoroborate, 1-ethyl-3-butylimidzolium tetrafluoroborate, 1-ethyl-2,3-dimethylimidazolium tetrafluoroborate, 1-ethyl-3,5-dimethylimidazolium tetrafluoroborate, 1,3-diethyl-5-methylimidazolium tetrafluoroborate, and 1-ethylimidazolium tetrafluoroborate; an alkyl-substituted imidazolium chloride wherein the above tetrafluoroborate anion is replaced with a chloride anion; an alkyl-substituted imidazolium bromide wherein the above tetrafluoroborate anion is replaced with a bromide anion; an alkyl-substituted imidazolium iodide wherein the above tetrafluoroborate anion is replaced with an iodide anion; an alkyl-substituted imidazolium hexafluorophosphate wherein the above tetrafluoroborate anion is replaced with a tetrafluorophosphate anion; an alkyl-substituted imidazolium bis(perfluoroalkanesufonyl)amide wherein the above tetrafluoroborate anion is replaced with a bis(perfluoroalkanesulfonyl)amide anion; an alkyl-substituted imidazolium alkylcarboxylate wherein the above tetrafluoroborate anion is replaced with an alkylcarboxylate anion; and an alkyl-substituted imidazolium alkanesulfonate wherein the above tetrafluoroborate anion is replaced with an alkanesulfonate anion.

Examples of the alkyl-substituted pyridinium salt include salts which are composed of a pyridinium cation, of which at least one nitrogen atom on the pyridine ring is bonded to the above-mentioned optionally substituted alkyl group, and the above-mentioned anionic species. Examples thereof include an alkyl-substituted pyridinium tetrafluoroborate such as N-methylpyridinium tetrafluoroborate, N-ethylpyridinium tetrafluoroborate, N-propylpyridinium tetrafluoroborate, N-butylpyridinium tetrafluoroborate, N-butyl-4-methylpyridinium tetrafluoroborate, N-isobutylpyridinium tetrafluoroborate, and N-pentylpyridinium tetrafluoroborate; an alkyl-substituted pyridinium chloride wherein the above tetrafluoroborate anion is replaced with a chloride anion; an alkyl-substituted pyridinium bromide wherein the above tetrafluoroborate anion is replaced with a bromide anion; an alkyl-substituted pyridinium iodide wherein the above tetrafluoroborate anion is replaced with an iodide anion; an alkyl-substituted pyridinium hexafluorophosphate wherein the above tetrafluoroborate anion is replaced with a tetrafluorophosphate anion; an alkyl-substituted pyridinium bis(perfluoroalkanesufonyl)amide wherein the above tetrafluoroborate anion is replaced with a bis(perfluoroalkanesulfonyl)amide anion; an alkyl-substituted pyridinium alkylcarboxylate wherein the above tetrafluoroborate anion is replaced with an alkylcarboxylate anion; and an alkyl-substituted pyridinium alkanesulfonate wherein the above tetrafluoroborate anion is replaced with an alkanesulfonate anion.

Examples of the quaternary ammonium salt include salts composed of the ammonium cations, which are composed of four above-mentioned optionally substituted alkyl groups, which may be the same or different, and nitrogen atoms, and the above-mentioned anionic species. Examples thereof include a quaternary ammonium tetrafluoroborate such as trimethylpentylammonium tetrafluoroborate, trimethylhexylammonium tetrafluoroborate, trimethylheptylammonium tetrafluoroborate, trimethyloctylammonium tetrafluoroborate and triethylpentylammonium tetrafluoroborate; a quaternary ammonium hexafluorophosphate wherein the above tetrafluoroborate anion is replaced with a tetrafluorophosphate anion; a quaternary ammonium bis(perfluoroalkanesufonyl)amide wherein the above tetrafluoroborate anion is replaced with a bis(perfluoroalkanesulfonyl)amide anion; a quaternary ammonium alkylcarboxylate wherein the above tetrafluoroborate anion is replaced with an alkylcarboxylate anion; and a quaternary ammonium alkanesulfonate wherein the above tetrafluoroborate anion is replaced with an alkanesulfonate anion.

Examples of the quaternary phosphonium salt include salts composed of the ammonium cations which are composed of four above-mentioned optionally substituted alkyl groups, which may be the same or different, and phosphorus atoms, and the above-mentioned anionic species. Examples thereof include a quaternary phosphonium tetrafluoroborate such as trimethylpentylphosphonium tetrafluoroborate and tetrabutylphosphonium tetrafluoroborate; a quaternary phosphonium hexafluorophosphate wherein the above tetrafluoroborate anion is replaced with a hexafluorophosphate anion; a quaternary phosphonium bis(perfluoroalkanesufonyl)amide wherein the above tetrafluoroborate anion is replaced with a bis(perfluoroalkanesulfonyl)amide anion; a quaternary phosphonium alkylcarboxylate wherein the above tetrafluoroborate anion is replaced with an alkylcarboxylate anion; a quaternary phosphonium alkanesulfonate wherein the above tetrafluoroborate anion is replaced with an alkanesulfonate anion.

Examples of the tertiary sulfonium salt include salts composed of the sulfonium cations, which are composed of three above-mentioned optionally substituted alkyl groups, which may be the same or different, and sulfur atoms, and the above-mentioned anionic species. Examples thereof include, for example, a tertiary sulfonium tetrafluoroborate such as triethylsulfonium tetrafluoroborate, tributylsulfonium tetrafluoroborate and tripropylsulfonium tetrafluoroborate; a tertiary sulfonium hexafluorophosphate wherein the above tetrafluoroborate anion is replaced with a hexafluorophosphate anion; a tertiary sulfonium bis(perfluoroalkanesufonyl)amide wherein the above tetrafluoroborate anion is replaced with a bis(perfluoroalkanesulfonyl)amide anion; a tertiary sulfonium alkylcarboxylate wherein the above tetrafluoroborate anion is replaced with an alkylcarboxylate anion; a tertiary sulfonium alkanesulfonate wherein the above tetrafluoroborate anion is replaced with an alkanesulfonate anion.

The hydrogenating temperature is usually 50 to 200°C, preferably 100 to 200°C and more preferably 130 to 180°C.

When the metal catalyst or the solvent contains water, it is preferred that the benzyl alcohol compound (2) is previously mixed with the metal catalyst and the solvent to remove water from the obtained mixture followed by contacting with the hydrogenating agent.

Examples of the method of removing water include a method comprising heating the mixture under reduced pressure, and a method comprising conducting azeotropic dehydration using an organic solvent making an azeotropic mixture with water such as toluene and xylene.

The progress of the reaction can be checked by a conventional analytical means such as gas chromatography, high performance liquid chromatography and the like.

After completion of the reaction, the tetrafluorotoluene compound (1) can be isolated, for example, by removing an insoluble matters such as the metal catalyst from the reaction mixture, adding water and as necessary a water-insoluble organic solvent to wash followed by concentrating the obtained organic layer. Examples of the water-insoluble organic solvent include aromatic hydrocarbon solvents such as toluene, xylene and chlorobenzene; aliphatic hydrocarbon solvents such as pentane, hexane and heptane; halogenated hydrocarbon solvents such as dichloromethane, dichloroethane and chloroform; ether solvents such as dethyl ether and methyl tert-butyl ether; and ester solvents such as ethyl acetate. When an ionic liquid having poor compatibility with water is used as the solvent, the aromatic hydrocarbon solvent or the aliphatic hydrocarbon solvent is preferably used.

When an ionic liquid having high compatibility with water is used as the solvent, an aqueous layer usually contains the ionic liquid, and the ionic liquid can be recovered by concentrating the aqueous layer. When an ionic liquid having poor compatibility with water is used as the solvent, the mixture is usually separated to three layers of an aqueous layer, an ionic liquid layer and an organic layer and the ionic liquid can be recovered by separating the ionic layer. The recovered ionic layer can be used again for the above-mentioned hydrogenating reaction of the benzyl alcohol compound (2).

Alternatively, the insoluble matters containing the metal catalyst, which is removed from the reaction mixture, can be used again for the above-mentioned hydrogenating reaction of the benzyl alcohol compound (2) according to circumstances, as it is or after washing with an organic solvent, water, an acid or a base.

The tetrafluorotoluene compound (1) obtained may be further purified by a conventional purification means such as distillation, column chromatography and the like.

4-Methyl-2,3,5,6-tetrafluorobenzyl alcohol can be produced by contacting the tetrafluorotoluene compound (1) with an aqueous solution of sulfuric acid.

As the tetrafluorotoluene compound (1), the reaction mixture containing the tetrafluorotoluene compound (1), which is obtained by the above-mentioned hydrating reaction of the benzyl alcohol compound (2) may be used as it is.

The content of sulfuric acid in the aqueous solution of sulfuric acid is usually 60 to 95% by weight, and preferably 80 to 95% by weight. The used amount of the aqueous solution of sulfuric acid is usually 1 mole or more as sulfuric acid per 1 mole of the tetrafluorotoluene compound (1), and while there is no specific upper limit, if it is too much, it is economically disadvantageous, and therefore, practical amount is 1 to 50 moles per 1 mole of the tetrafluorotoluene compound (1).

While the contact of the tetrafluorotoluene compound (1) and an aqueous solution of sulfuric acid is usually conducted in the absence of a solvent, it may be conducted in the presence of an organic solvent. Examples of the organic solvent include aromatic hydrocarbon solvents such as toluene, xylene and chlorobenzene; aliphatic hydrocarbon solvents such as pentane, hexane and heptane; and halogenated hydrocarbon solvents such as dichloromethane, dichloroethane and chloroform. The used amount of the organic solvent is usually 1 part by weight or less per 1 part of the tetrafluorotoluene compound (1).

The contacting temperature is usually 0 to 100°C and preferably 30 to 80°C.

While the tetrafluorotoluene compound (1) is usually contacted with the aqueous solution of sulfuric acid under a normal pressure, the tetrafluorotoluene compound (1) may be contacted with the aqueous solution of sulfuric acid under pressure.

The progress of the reaction can be checked by a conventional analytical means such as gas chromatography, high performance liquid chromatography and the like.

After completion of the contact, 4-methyl-2,3,5,6-tetrafluorobenzyl alcohol can be isolated, for example, by mixing the obtained reaction mixture with water and as necessary a water-insoluble organic solvent, leaving the resultant mixture at rest, separating an organic layer and then, concentrating the organic layer obtained. Examples of the water-insoluble organic solvent include the same as described above. 4-Methyl-2,3,5,6-tetrafluorobenzyl alcohol obtained may be further purified by a conventional purification means such as distillation, column chromatography and the like.

4-Methyl-2,3,5,6-tetrafluorobenzyl acetate can be produced by reacting the tetrafluorotoluene compound (1) with acetic anhydride in the presence of a metal triflate.

As the tetrafluorotoluene compound (1), the reaction mixture containing the tetrafluorotoluene compound (1), which is obtained by the above-mentioned hydrating reaction of the benzyl alcohol compound (2) may be used as it is.

The used amount of acetic anhydride is usually 1 mole or more per 1 mole of the tetrafluorotoluene compound (1). There is no specific upper limit, and while a large excess amount of acetic anhydride may be used as a solvent, it is practically 1 to 20 moles per 1 mole of the tetrafluorotoluene compound (1).

Examples of the metal triflate include aluminum triflate, tin triflate, cupper triflate, zinc triflate, scandium triflate, yttrium triflate, cerium triflate, ytterbium triflate and samarium triflate. A lanthanoid metal triflate such as cerium triflate, ytterbium triflate and samarium triflate is preferable, and ytterbium triflate is more preferable. While a commercially available metal triflate is usually used, one prepared by a known method may be used.

The used amount of the metal triflate is usually 0.01 to 0.5 mole per 1 mole of the tetrafluorotoluene compound (1).

The reaction of the tetrafluorotoluene compound (1) and acetic anhydride may be conducted in the absence of a solvent and may be in the presence of a solvent as described above. Alternatively, the reaction may be conducted in the presence of an inert organic solvent on the reaction. Examples of the inert organic solvent on the reaction include aliphatic hydrocarbon solvents such as pentane, hexane and heptane; and halogenated hydrocarbon solvents such as dichloromethane, dichloroethane and chloroform. The used amount of the inert organic solvent on the reaction is usually 1 part by weight or less per 1 part of the tetrafluorotoluene compound (1).

The reaction temperature is usually 0 to 200°C and preferably 50 to 150°C.

The reaction of the tetrafluorotoluene compound (1) and acetic anhydride is usually conducted by mixing the tetrafluorotoluene compound (1), acetic anhydride and the metal triflate to heat at the predetermined temperature, and the mixing order is not particularly limited.

While the reaction is usually conducted under a normal pressure, it may be conducted under pressure. The progress of the reaction can be checked by a conventional analytical means such as gas chromatography, high performance liquid chromatography and the like.

After completion of the reaction, 4-methyl-2,3,5,6-tetrafluorobenzyl acetate can be isolated, for example, by mixing the reaction mixture with water and as necessary a water-insoluble organic solvent, leaving the resultant mixture at rest, separating an organic layer and then, concentrating the organic layer obtained. Examples of the water-insoluble organic solvent include the same as described above. 4-Methyl-2,3,5,6-tetrafluorobenzyl acetate obtained may be further purified by a conventional purification means such as distillation, column chromatography and the like.

4-Methyl-2,3,5,6-tetrafluorobenzyl bromide can be produced by reacting the tetrafluorotoluene compound (1) with a brominating agent.

As the tetrafluorotoluene compound (1), the reaction mixture containing the tetrafluorotoluene compound (1), which is obtained by the above-mentioned hydrating reaction of the benzyl alcohol compound (2) may be used as it is.

Examples of the brominating agent include a bromine compound showing Lewis acidity such as boron tribromide, aluminum tribromide and phosphorus tribromide, and hydrogen bromide.

The used amount of the brominating agent is usually 1 to 5 moles per 1 mole of the tetrafluorotoluene compound (1).

The reaction of the tetrafluorotoluene compound (1) and the brominating agent is usually conducted in the presence of a solvent. The solvent is not particularly limited in so far as it is an inert organic solvent on the reaction. Examples thereof include aromatic hydrocarbon solvents such as toluene, xylene and chlorobenzene; aliphatic hydrocarbon solvents such as pentane, hexane and heptane; and halogenated hydrocarbon solvents such as dichloromethane, dichloroethane and chloroform. While the used amount thereof is not particularly limited, the practical used amount thereof is 50 parts by weight or less per 1 part of the tetrafluorotoluene compound (1) in consideration of volume efficiency.

The reaction temperature is usually -30 to 50°C.

The reaction is usually conducted by mixing the tetrafluorotoluene compound (1), the brominating agent and as necessary the inert organic solvent on the reaction to adjust the resultant mixture at the predetermined temperature. While the mixing order is not particularly limited, the brominating agent is preferably added to a mixture of the tetrafluorotoluene compound (1) and the inert organic solvent on the reaction.

While the reaction is usually conducted under a normal pressure, it may be conducted under pressure. The progress of the reaction can be checked by a conventional analytical means such as gas chromatography, high performance liquid chromatography and the like.

After completion of the reaction, an organic layer containing 4-methyl-2,3,5,6-tetrafluorobenzyl bromide can be obtained, for example, by mixing the reaction mixture with water or a basic aqueous solution and as necessary mixing with a water-insoluble organic solvent followed by separating, and 4-methyl-2,3,5,6-tetrafluorobenzyl bromide can be isolating by concentrating the organic layer. Examples of the water-insoluble organic solvent include the same as described above. 4-Methyl-2,3,5,6-tetrafluorobenzyl bromide obtained may be further purified by a conventional purification means such as distillation, column chromatography and the like.

### Examples

The present invention is illustrated by Examples in more detail below, but the present invention is not limited to these Examples. The analysis was conducted by gas chromatography area percentage method.

### Example 1

Into a 50 ml flask equipped with a reflux condenser, 5 g of slurry of sponge cobalt (commercially available from Aldrich, Raney (a registered trade mark of W. R. Grace) 2700 Cobalt), 5 g of 4-methoxymethyl-2,3,5,6-tetrafluorobenzyl alcohol and 5 g of 1-methyl-4-butylimidazolium tetrafluoroborate were charged. After removing water from the obtained mixture at 8 kPa and at an inner temperature of 80°C, a gas phase part in the flask was substituted with nitrogen. Further, the gas phase part was substituted with hydrogen and a 1L-volume rubber balloon full filed with hydrogen was equipped with the flask. The mixture was heated to 160°C to effect reaction at 160°C for 4 hours. After completion of the reaction, the reaction mixture was cooled to room temperature and 20 g of hexane and 20 g of water were added thereto. After removing an insoluble matters by filtration from the mixture obtained, the filtrate obtained was left at rest and separated to an organic layer and an aqueous layer. The aqueous layer was extracted twice with 20 g of hexane. The hexane layers were mixed with the previous obtained organic layer to concentrate to obtain 4.5 g of a pale yellow oil containing 4-methoxymethyl-2,3,5,6-tetrafluorotoluene. The purity was 98% and the yield was 95%. Alternatively, 4-methoxymethyl-2,3,5,6-tetrafluorobenzyl alcohol was recovered in 3%. GC-MS:M⁺=208
¹H-NMR (δₚₚₘ, CDCl₃, TMS standard): 2.29 (s, 3H), 3.39 (s, 3H), 4.56 (s, 2H)

### Example 2

The reaction was conducted according to a same manner as that of Example 1, except that the used amount of slurry of cobalt sponge was 4.5 g and the reaction time was 8 hours, to obtain 4.6 g of a pale yellow oil containing 4-methoxymethyl-2,3,5,6-tetrafluorotoluene. The purity was 99% and the yield was 98%. Alternatively, 4-methoxymethyl-2,3,5,6-tetrafluorobenzyl alcohol was recovered in 2%.

### Example 3

Into a 50 ml flask equipped with a reflux condenser, 900 mg of slurry of sponge cobalt (commercially available from Aldrich, Raney (a registered trade mark of W. R. Grace) 2700 Cobalt), 1.12 g of 4-methoxymethyl-2,3,5,6-tetrafluorobenzyl alcohol and 5 g of trioctylmethylammonium bis(trifluoromethanesulfonyl)imide were charged. After removing water from the obtained mixture at 8 kPa and at an inner temperature of 80°C, a gas phase part in the flask was substituted with nitrogen. Further, the gas phase part was substituted with hydrogen and a 1L-volume rubber balloon full filed with hydrogen was equipped with the flask. The mixture was heated to 150°C to effect reaction at 150°C for 5 hours. After completion of the reaction, the reaction mixture was cooled to room temperature and 5 g of hexane and 10 g of water were added thereto. After removing an insoluble matters by filtration from the mixture obtained, the filtrate obtained was left at rest and separated to an organic layer, an aqueous layer and an ionic liquid layer. The ionic liquid layer was extracted twice with 5 g of hexane. The hexane layers were mixed with the previous obtained organic layer to concentrate to obtain 916 mg of a pale yellow oil containing 4-methoxymethyl-2,3,5,6-tetrafluorotoluene. The purity was 98% and the yield was 86%. Alternatively, 4-methoxymethyl-2,3,5,6-tetrafluorobenzyl alcohol was recovered in 4%.

### Example 4

Into a 200 ml pressure-resistant reaction tube, 4.5 g of slurry of sponge cobalt (commercially available from Aldrich, Raney (a registered trade mark of W. R. Grace) 2700 Cobalt), 5 g of 4-methoxymethyl-2,3,5,6-tetrafluorobenzyl alcohol and 15 g of toluene were charged. After a gas phase part in the reaction tube was substituted with nitrogen, the gas phase part was substituted twice with hydrogen at 0.4 MPa. The hydrogen pressure in the reaction tube was adjusted to a gauge pressure of 0.4 MPa, and then, the tube was heated to 150°C to effect reaction at 150°C for 18 hours. During this period, the gauge pressure was increased up to 0.8 MPa and then decreased to 0.6 MPa. After completion of the reaction, the reaction mixture was cooled to room temperature and 10 g of water was added thereto. After removing an insoluble matters by filtration from the mixture obtained, the insoluble matters were washed with 10 g of toluene. The wash liquid obtained was mixed with the previously obtained filtrate, leaving at rest followed by separating an organic layer. The organic layer was concentrated to obtain 4.7 g of a pale yellow oil containing 4-methoxymethyl-2,3,5,6-tetrafluorotoluene. The purity was 94% and the yield was 95%.

### Example 5

Into a 50 ml flask equipped with a reflux condenser, 900 mg of slurry of sponge cobalt (commercially available from Aldrich, Raney (a registered trade mark of W. R. Grace) 2700 Cobalt), 1 g of 4-isopropoxymethyl-2,3,5,6-tetrafluorobenzyl alcohol and 3 g of 1-methyl-4-butylimidazolium tetrafluoroborate were charged. After removing water from the obtained mixture at 8 kPa and at an inner temperature of 80°C, a gas phase part in the flask was substituted with nitrogen. Further, the gas phase part was substituted with hydrogen and a 1L-volume rubber balloon full filed with hydrogen was equipped with the flask. The mixture was heated to 160°C to effect reaction at 160°C for 6 hours. After completion of the reaction, the reaction mixture was cooled to room temperature and 5 g of hexane and 10 g of water were added thereto. The mixture obtained was left at rest followed by separating to an organic layer and an aqueous layer. The aqueous layer was extracted twice with 5 g of hexane. The hexane layers were mixed with the previous obtained organic layer to concentrate to obtain 980 mg of a pale yellow oil containing 4-isopropoxymethyl-2,3,5,6-tetrafluorotoluene. The pale yellow oil was analyzed by gas chromatography mass analysis to find out that it was a mixture of 4-isopropoxymethyl-2,3,5,6-tetrafluorotoluene (M⁺=236) and 4-isopropoxymethyl-2,3,5,6-tetrafluorobenzyl alcohol. The yield of 4-isopropoxymethyl-2,3,5,6-tetrafluorotoluene was 14% and 4-isopropoxymethyl-2,3,5,6-tetrafluorobenzyl alcohol was recovered in 85%.

### Example 6

Into a 50 ml flask, 190 mg of the oil containing 4-methoxymethyl-2,3,5,6-tetrafluorotoluene, which was obtained in the above-mentioned Example 1, and 2 g of 90% sulfuric acid were charged and the resultant mixture was heated to 50°C and kept while stirring at 50°C for 30 minutes. After the reaction, the mixture was cooled to room temperature and diluted with 10g of water and then, 5 g of ethyl acetate was added thereto. The organic layer obtained was analyzed by a gas chromatography internal standard method to find out that the yield of 4-methoxymethyl-2,3,5,6-tetrafluorobenzyl alcohol was 85%.

### Example 7

Into a 50 ml flask, 200 mg of the oil containing 4-methoxymethyl-2,3,5,6-tetrafluorotoluene, which was obtained in the above-mentioned Example 1, and 2 g of chloroform were charged and the obtained mixture was adjusted at 20°C. To the mixture, a mixed solution of 750 mg of boron tribromide and 2 g of chloroform was added dropwise at 20°C over 30 minutes to effect reaction for 1 hour. After completion of the reaction, 10 g of water was added to the reaction mixture while cooling with an ice to obtain a chloroform layer containing 4-methoxymethyl-2,3,5,6-tetrafluorobenzyl bromide and an aqueous layer.
The yield was 94%.

### Example 8

Into a 50 ml flask, 104 mg of the oil containing 4-methoxymethyl-2,3,5,6-tetrafluorotoluene, which was obtained in the above-mentioned Example 1, 1 g of acetic anhydride and 100 mg of ytterbium triflate were charged and the obtained mixture was reacted at 140°C for 1 hour. After completion of the reaction, 5 g of ethyl acetate and 5 g of water were added to the reaction mixture to obtain an ethyl acetate layer containing 4-methoxymethyl-2,3,5,6-tetrafluorobenzyl acetate and an aqueous layer. The yield was 87%.

### Industrial Applicability

A tetrafluorotoluene compound represented by the formula (1) of the present invention is a novel compound and it can be derived to 4-methyl-2,3,5,6-tetrafluorobenzyl alcohol, 4-methyl-2,3,5,6-tetrafluorobenzyl acetate and 4-methyl-2,3,5,6-tetrafluorobenzyl bromide, which are useful as synthetic intermediates of pharmaceuticals and agrichemicals.

## Claims

**1.** A tetrafluorotoluene compound represented by the formula (1): 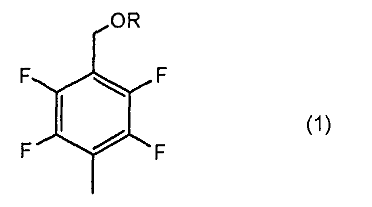 wherein R represents a C1-C6 alkyl group.

**2.** The tetrafluorotoluene compound according to claim 1, wherein R is a methyl group.

**3.** A method for producing a tetrafluorotoluene compound represented by the formula (1): 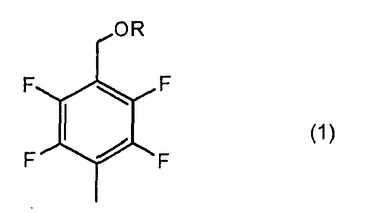 wherein R represents a C1-C6 alkyl group, which comprises hydrogenating a tetrafluorobenzyl alcohol compound represented by the formula (2): 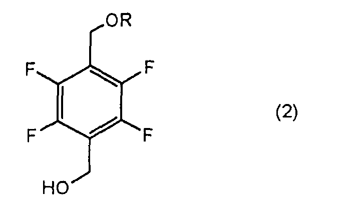 Wherein R represents the same as defined above.

**3.** The method according to claim 2, wherein the tetrafluorobenzyl alcohol compound represented by the formula (2) is hydrogenated using hydrogen.

**4.** The method according to claim 3, wherein the tetrafluorobenzyl alcohol compound represented by the formula (2) is hydrogenated in the presence of a metal catalyst.

**5.** The method according to claim 4, wherein the metal catalyst is sponge cobalt.

**6.** The method according to any one of claims 3 to 5, wherein the reaction is conducted in the presence of an ionic liquid.

**7.** The method according to claim 6, wherein the ionic liquid is an alkyl-substituted imidazolium salt or a quaternary ammonium salt.

**8.** A method for producing 4-methyl-2,3,5,6-tetrafluorobenzyl alcohol comprising contacting tetrafluorotoluene represented by the represented by the formula (1): 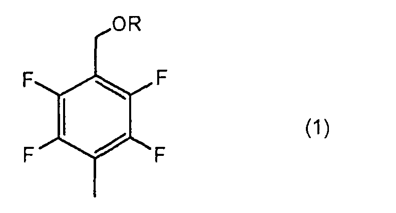 wherein R represents a C1-C6 alkyl group, with an aqueous solution of sulfuric acid.

**9.** The method according to claim 8, wherein the content of sulfuric acid in the aqueous solution of sulfuric acid is 60 to 95% by weight.

**10.** A method for producing 4-methyl-2,3,5,6-tetrafluorobenzyl acetate comprising reacting a tetrafluorotoluene compound represented by the represented by the formula (1): 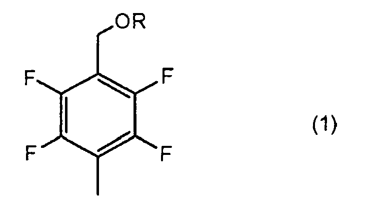 wherein R represents a C1-C6 alkyl group, with acetic anhydride in the presence of a metal triflate.

**11.** The method according to claim 10, wherein the metal triflate is a lanthanoid metal triflate.

**12.** A method for producing 4-methyl-2,3,5,6-tetrafluorobenzyl bromide comprising reacting a tetrafluorotoluene compound represented by the represented by the formula (1): 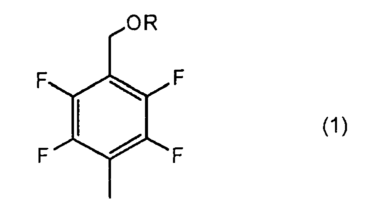 wherein R represents a C1-C6 alkyl group, with a brominating agent.

**13.** The method according to claim 12, wherein the brominating agent is boron tribromide.

**14.** The method according to claim 8, wherein the tetrafluorotoluene compound represented by the formula (1) is a tetrafluorotoluene compound obtained by hydrogenating a tetrafluorobenzyl alcohol compound represented by the formula (2).

**15.** The method according to claim 10, wherein the tetrafluorotoluene compound represented by the formula (1) is a tetrafluorotoluene compound obtained by hydrogenating a tetrafluorobenzyl alcohol compound represented by the formula (2).

**16.** The method according to claim 12, wherein the tetrafluorotoluene compound represented by the formula (1) is a tetrafluorotoluene compound obtained by hydrogenating a tetrafluorobenzyl alcohol compound represented by the formula (2).
